# EUROPEAN PATENT SPECIFICATION

(11) **EP 1 359 228 B1**
(45) Date of publication and mention of the grant of the patent: **27.11.2013**
(21) Application number: 02354064.4
(22) Date of filing: 23.04.2002
(51) Int. Cl.: C12Q 1/68, B01L 3/00

(54) **DNA authentification based on scattered-light detection**
Nukleinsäure authentifikation mittels detektion des streulichtes
Authentification d'acide nucléique selon la détection de la lumière diffusée

(43) Date of publication of application: 05.11.2003
(73) Proprietor: Accenture Global Services Limited, Dublin 4 (IE)
(72) Inventor: Pelletier, Olivier, 06600 Antibes (FR)
(74) Representative: Thibon, Laurent

(56) References cited:
- EP-A- 0 288 737
- EP-A- 0 317 074
- EP-A1- 0 239 318
- WO-A-00/71243
- WO-A-01/38865
- WO-A-01/85341
- WO-A-01/92580
- US-A- 4 801 550
- US-A1- 2001 055 812

## Description

### FIELD OF THE INVENTION

The present invention relates to a device and a method for determining whether two DNA sequences are identical.

### BACKGROUND

A comparison between two DNA molecules may be useful in many applications. For example, a well-known application is determining whether the DNA of a suspect was present at the scene of a crime by comparing a DNA sequence with another sequence found at the crime location. Indeed, DNA molecules can be found and in any biological substance from a person (for example, a small quantify of saliva or sperm or a single hair). Very small samples contain enough DNA for this type of comparative analysis to be performed.

Another example of this type of application concerns the authentication of valuable objects (for example, a painting or any other art object). In such an application, synthetic DNA is generally utilized. A particular DNA sequence is included, for example, in the paint. Then, many years later, it is possible to compare the DNA molecule embedded in the paint with a reference molecule to authenticate the painting.

As human genetic materials differ from each other essentially by single mutations at well defined positions, e.g. (SNPs), the actual implementation of this type of approach in forensic science generates a list of the values of the nucleotides at these positions and these lists are compared (see DNA Technology in Forensic Science by the National Res. Council, ed.) A major drawback is that it is necessary to obtain the list of nucleotides for a test sequence to be compared to the reference list. Such an analysis is long and complex.

Another known technique uses the Watson-Crick complement strand of a reference sample covalently bound to fluorescent markers and a strand of the sample to be compared. If there is identity of the samples, the strands "stick" or hybridize together to form a double helix. The fluorescent markers serve to identify the presence of the double helix. Such a principle is extensively used in the DNA microarrays used for monitoring gene expression levels (see for example: DNA Microarrays: A Practical Approach by M. Schena, ed.)

A drawback of this technique is the compulsory use of fluorescent markers. Indeed, such markers need precautionnal storage and use to remain efficient. Further, fluorescent markers are very expensive.

EP 0 288 737 describes a hybridisation method for detecting a target polynucleotide in a test medium characterized by the use of water suspensible microparticles and change in turbidity.

US 4 801 550 discloses a method for separately recovering nucleic acids present in a sample containing a mixture of nucleic acids using solvents.

WO 01/92580 discloses a method of preparing a composition comprising un-aggregated nucleic acid complexes. The turbidity is used as a parameter for determining properties like shape.

EP 0 317 074 discloses a method producing a conjugate of an insoluble particle and a hybridized target nucleic acid and separating the conjugate from any excess probe and/or primer by filtration and washing.

European patent application 0 239 318 describes an assay method using a filter of pore size sufficient to allow particles to pass through.

There is thus a tremendous need to develop a simple and inexpensive method and device for determining whether two DNA sequences are identical.

### SUMMARY OF THE INVENTION

In order to meet these needs, the present invention is directed to a device and a method for assessing the identity between a reference DNA sequence and the sequence of a test DNA sample. The DNA sequence of the reference DNA is not necessarily known, but it is assumed that the practitioner has access to a sample of single stranded DNA with a sequence complementary to the reference sequence. This sample is referred to herein as the reference sample. The test sample is also single stranded. Both the test and the reference DNA samples are isolated and made singled stranded utilizing standard procedures (see Sambrook, et al. Molecular Cloning: A Laboratory Manual).

According to the present invention, DNA molecules from the reference sample are attached to a solid support. Then a solution containing the test DNA sample is added. The DNA samples are allowed to hybridize under conditions such that the two strands will hybridize only if they have greater than approximately 95% identity. The test DNA sample hybridizes with the reference DNA sample and then remains attached to the solid support. If the DNA sequences are less than approximately 95 to 100% identical, there is no hybridization, and the test strands remain in the solution.

Any solid support allowing single stranded DNA molecules to be attached to it can be used. For example, magnetic micro-particles or latex micro-particles can be used. Another example can be microbeads like the Streptavidin MagneSphere® Paramagnetic Particles from Promega Corporation, Madison, Wisconsin.

The non-solubility of DNA in a set of given solvents is utilized to determine whether the test and reference sequences are at least approximately 95 to 100% identical. Having filtered the mixture of the preceding step with a filter having a characteristic size adapted to retain the solid support but to permit single stranded DNA molecules to pass through the filter, a solvent (for example, ethanol, acetone) is added to the filtered solution. If DNA is present, the DNA will precipitate upon addition of the solvent. This occurs when the test and reference sequences are different. If DNA is not present in the filtered solution, the solvent mixture will remain clear (transparent) because there is no DNA to precipitate upon addition of the solvent. This occurs when the test and reference sequences are approximately 95 to 100% identical (the DNA molecules of the test sample have hybridized with the molecules from the reference sample). In sum, if the test and reference DNA samples are identical, the test sample remains bound to the reference sample and cannot pass through the filter. If DNA does not pass through the filter, there is no DNA to precipitate upon addition of the solvent. If the test DNA sequence is different, it does not hybridize to the reference DNA, but instead passes through the filter where it can be precipitated with solvent and detected.

According to the present invention, to detect DNA it is then sufficient to test the light scattering or turbidity of the resulting filtered solution. By detecting the turbidity of the filtered mixture, one can determine whether or not the sample of DNA introduced in an analyzing chamber already containing a reference sample is 95-100% identical to the reference sample.

Preferably, the turbidity of the filtered solution added to the solvent is compared to the turbidity of a control sample made of a solution containing only the DNA molecules to be tested and the solvent. Then, the control sample should have a high turbidity resulting from the presence of aggregates. It must be different from the turbidity of the solution mixture if the DNA sequence to be compared is identical to the reference sequence. This format alleviates a false detection in the case the sample to be tested does not contain any DNA.

The present invention is thus directed to a method for determining whether or not the sequence of a test DNA molecule and the sequence of a reference DNA molecule are 95 to 100% identical. The method of the invention includes the steps of: a) preparing a first solution of single stranded test DNA molecules; b) attaching a reference DNA molecule to a solid support to form a solid support-reference DNA complex; c) hybridizing the single stranded test DNA molecules with the solid support-reference DNA complex to form a solid support-reference DNA-test DNA complex wherein the solid support-reference DNA-test DNA complex is formed when the reference DNA and the test DNA sequences are 95 to 100% identical; d) filtering the solid support-reference DNA-test DNA complex through a filter under conditions such that said test DNA molecules passes through said filter into a filtered solution if the test DNA has not hybridized to said reference DNA, the filter having a cut-off size chosen to prevent the flow of the solid support but allowing the flow of free DNA solution; e) adding a solvent to the filtered solution to form solvent treated filtered solution; f) detecting the presence or absence of the test DNA in said solvent treated filtered solution by measuring the opacity or turbidity or the solvent treated filtered solution wherein the presence of DNA is the filtered solution indicates that the sequences of the test and reference DNA samples were less than 95% identical and wherein the absence of DNA is the filtered solution indicates that the sequences of the test and reference DNA samples were more than 95% identical.

In the method of the invention the solvent may be selected ethanol or acetone. In one format, the opacity or turbidity is measured by shining light through said solvent treated filtered solution. The light may be a laser light

As discussed above, in the method of the invention, the solid support may be a suspension of streptavidin-coated microbeads, and the reference DNA molecules may include a biotin group.

The present invention is further directed to a device (20) for implementing the above method. The device may include an injection chamber arranged to receive the test solution containing single stranded test DNA molecules; a hybridization chamber hybridizing the single stranded test DNA molecules to the reference DNA molecules attached to the solid support to generate the solid support-reference DNA-test DNA complex, an output of the injection chamber being connected to an input of the hybridization chamber ; a filter having a cut-off size chosen to prevent flow of the solid support but allowing the flow of free DNA solution, the filter being arranged to filter the content of the hybridization chamber; a detection chamber for receiving the content of the hybridization chamber after said filtering; and a photodiode unit arranged to record the light intensity scattered by the indicator solution in the detection chamber to detect the presence or absence of test DNA.

The device may also include a light source (30), preferably a laser source, to illuminate the indicator solution contained in the detection chamber.

In the device, the detection chamber may include a first compartment to contain the indicator solution outputted from the hybridization chamber, and a second compartment to contain a control solution of the DNA solution to be tested.

### DESCRIPTION OF THE DRAWINGS

The foregoing and other objects, features, aspects and advantages of the invention will become apparent from the following detailed description of embodiments, given by way of illustration and not of limitation with reference to the accompanying drawings.
Figure 1 is a schematic flowchart of a known DNA authentication process;
Figure 2 is a flowchart of an embodiment of the DNA authentication process of the present invention; and
Figure 3 represents, very schematically and functionally, an embodiment of a DNA authentication device according to the present invention.

### DETAILED DESCRIPTION OF THE INVENTION

In order to more fully understand the invention, the following definitions are provided:
As used herein a "reference sample" refers to a sample containing a reference DNA sequence.
A "test sample" refers to a sample to which the reference sample is compared.
"Reference DNA" refers to a DNA sequence in the reference sample that is compared to the test sequence in the test sample.
"Test DNA" refers to a sequence in the test sample that is compared to the reference DNA sequence in the reference sample.
"DNA Sequence Identify" refers to DNA sequences having at least approximately 95% identity between the component nucleotides.
"DNA extraction" refers to the operation whereby the DNA molecules to be tested are purified and isolated (and possibly amplified).

Taking into account these definitions, the present invention is directed a device and a method for determining if two DNA sequences are identical.

A DNA molecule is a linear assembly of nucleotides (Adenine, Cytosine, Guanine or Thymine). The order of these nucleotides can be arbitrary in the linear assembly. In nature, a DNA molecule constitutes a biologic identifiant. In synthetic form, a DNA molecule can be used to store information. Nucleotides A, C, G and T are pairwise complementary (C with G and A with T).

DNA molecules can be single stranded or double stranded. The conditions under which 2 single strands of DNA can combine to form double stranded DNA under a process known as hybridization are very well known (see Sambrook, et al. Molecular Cloning: A Laboratory Manual) DNA hybridization is at a maximum whenever the sequences of the 2 DNA strands are pairwise complementary along 100% of the sequence. Whenever mismatches occur between the 2 sequences, the amount of hybridization is reduced. Temperature and ionic strength and the length of the DNA molecules are all known to play a role: it is possible to limit hybridization to pair of molecules with a very small number of sequence mismatches (e.g. 25% by increasing temperature and ionic strength. Also, the longer the DNA molecules, the more restrictive are the hybridization conditions. For DNA molecule A with a given nucleotide sequence, it is possible to determine chemical conditions hybridization conditions under which the probability that another randomly chosen DNA molecule B will hybridize to A is extremely small, for example less than 5%. Unless otherwise stated, we will assume in the practice of the present invention hybridization reactions are performed under these hybridization conditions whenever dealing with the hybridization of DNA molecules. This means that, under such conditions, the 95 to 100% identity of two sequences SEQA and SEQB can be inferred by monitoring the hybridization of DNA molecules with sequence SEQA with DNA molecules whose sequence is complementary to SEQB. If the DNA molecules hybridize, then SEQA is 95 to 100% identical to SEQB, if not, they are different or less than 95% identical. This defines the notion of "identical sequences" for the present invention.

In this invention, we make use of the fact that single stranded-DNA molecules can be attached to solid supports. By attached, we mean that it is possible to create a permanent bond between the DNA molecule and the solid surface. Once attached to a solid support the lifetime of the attached DNA will be much greater than the timescale on which the present invention is intended to be used. This permanent bond can be a chemical bond if the end of the DNA molecule is functionalized in order to perform a chemical reaction with reactive groups present on the surface of the solid support. It can also be any sort of bond that will have the property that the DNA molecules won't be free to leave the molecular vicinity of the surface. For example, DNA molecules can be modified to provide a biotin group at end of the DNA molecule and the surface of the solid support can be coated with streptavidin groups. Because of the strong biotin/streptavidin interaction, under appropriate conditions, the DNA molecule will also be attached to the surface. It is essential to note that single stranded DNA molecules attached to a solid support in this manner are free to hybridize with single stranded, complementary, DNA molecules. If such hybridization occurs, both DNA strands will be attached to the solid surface, in the meaning that has been defined previously.

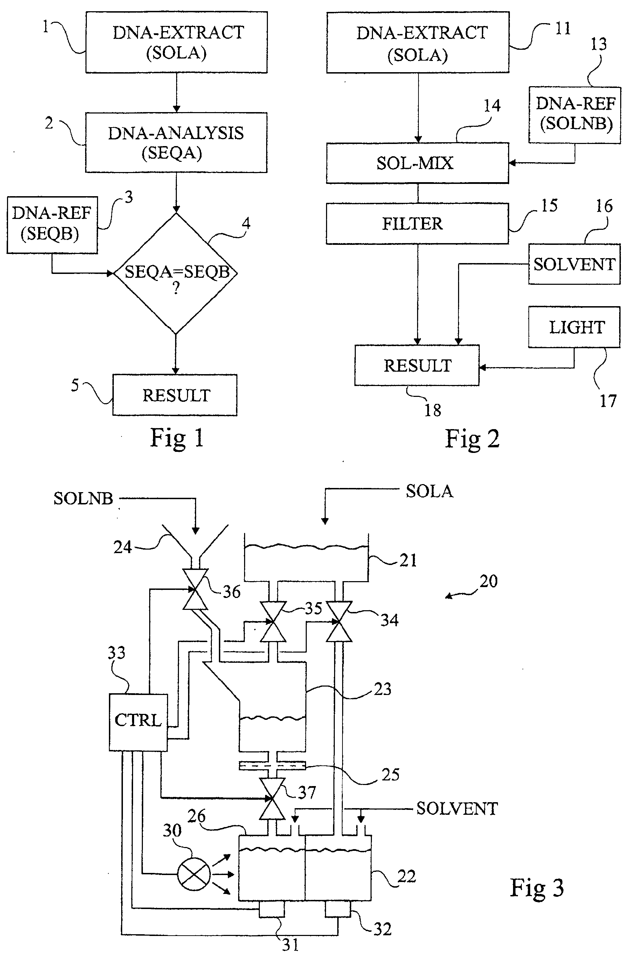

Figure 1 is a schematic flowchart of a conventional DNA authentication (comparison) process.

In a first step (block 1, DNA-EXTRACT) DNA molecules, and more precisely, strands of the DNA molecule to be compared are extracted in order to obtain a solution (SOLA) containing the DNA sequence.

The second step (block 2, DNA-ANALYSIS) includes in analyzing the DNA strand in order to obtain its DNA sequence (SEQA). At the end of this step, the list of the nucleotides codes (A, C, G, T) of the DNA sequence to be compared is known.

A reference DNA sequence (SEQB) is then extracted (block 3, DNA-REF), for instance, from a memory.

To determine the identity of the sequences SEQA and SEQB, the nucleotides lists are compared to each other (block 4, SEQA=SEQB?).

The comparison step 4 gives the result (block 5, RESULT) of the comparison.

Figure 2 is a flowchart illustrating an embodiment of the process according to the present invention.

The first step includes, as in a conventional process, extracting from a biological sample (hair, saliva, sperm, etc.) or from a synthetic sample, the DNA strand in a solution (block 11, DNA-EXTRACT). The DNA solution (SOLA) containing the test sample is to be compared to a reference sample of DNA.

According to the present invention, both DNA sequences may be compared in a soluble form.

A reference solution (SOLNB) containing the reference sample attached to a solid support such as silica or latex microbeads is then (or in parallel) prepared (DNA-REF, block 13). In one format, the microbeads are streptavidin-coated beads. In this format, the reference DNA strands have a biotin group at their end, allowing them to be attached to the streptavidin groups present on the surface of the solid surface.

The solutions SOLA and SOLNB are mixed (block 14, SOL-MIX). If the sequence of the test sample is 95 to 100% identical to the reference sequence, the test and reference strands will hybridize. This will ensure that the test molecules will in turn be attached to the solid surface. If not, the test DNA strands will remain free in the solution.

The mixture is then filtered (block 15, FILTER) to retain the solid surfaces (and the DNA strands attached thereto). The filtered solution contains DNA molecules from the test sample only if the test and reference sequences are not identical and hybridization did not take place.

A solvent (block 16, SOLVENT), for example, ethanol, acetone or any solvent in which DNA molecules are not soluble (a poor solvent), is added to the filtered solution. According to the invention, the solvent is used as a way to reveal the presence of DNA molecules in the filtered solution. If DNA molecules are present, they will aggregate to form large (i.e. size > 1 micron) aggregates.

Such aggregates modify the turbidity of the solution. Then, according to a preferred embodiment of the present invention, one will illuminate (block 17, LIGHT) a transparent container containing the solution to directly obtain the result (block 18, RESULT) of the comparison of the two DNA samples.

Preferably, the turbidity of the solution obtained after the filtration step is compared to the turbidity of a control sample made of solution A and only the solvent. Then, the control sample comprises aggregates and has a turbidity that is different from the turbidity of the filtered solution if the current DNA is identical to the reference DNA. Such a preferred embodiment alleviates a false detection in case the sample to be tested does not contain any DNA.

An advantage of the present invention is that the comparison of two DNA samples is very easy. In particular, according to the present invention, it is not necessary to analyze both sequences in order to obtain the complete list of nucleotides as in the conventional example of figure 1.

Compared to the use of fluorescent markers, the present invention has the advantage of eliminating the needs of such fluorescent markers.

Another advantage of the present invention is that the results are obtained very quickly compared to methods requiring DNA sequence analysis.

Another advantage of the present invention is that measuring the turbidity makes detection easier and less expensive. In particular, the sensor to detect the modification of turbidity does not need to be as sensitive as would be the case for a detection based on the measurement of the opacity.

Another advantage of the present invention is that its implementation is compatible with a miniaturization required to constitute a portable device. Furthermore, the invention could also be implemented as a microfluidic device.

This advantage will be better understood in connection with the description of an embodiment of a device according to the present invention made in connection with figure 3.

Figure 3 represents, schematically and functionally, an exemplary embodiment of a device 20 for comparing two DNA samples according to the present invention. First, DNA strands are extracted from any conventional source. Next, DNA solutions SOLA and SOLNB to be compared are introduced in the device 20 according to the present invention. In the example of figure 3, the DNA solution A is introduced in an injection chamber 21. The solution in chamber 21 is preferably divided into two parts. One part goes directly to a first compartment 22 of a turbidity detection chamber, which constitutes the control sample compartment according to a preferred embodiment. The other part goes through a hybridization chamber 23. The hybridization chamber 23 contains a solid support such as microbeads grafted with DNA molecules with a sequence complementary to the reference sequence B. Such grafted microbeads are obtained in a conventional way by using the appropriate chemical procedure to graft the DNA molecules to the microbeads, for example, such as described above for biotin/streptavidin. The actual chemical reaction to be carried depends on the active functional groups formed on the surface of the solid surface.

In Figure 3, the retention of the DNA reference molecules attached to the microbeads introduced in hybridization chamber 23 is illustrated in the form of a preparation chamber 24 in which are introduced to the solid surface and the DNA molecules (globally designated by SOLNB). Alternatively solution SOLNB may be prepared well in advance and stored in the hybridization chamber 23 or in a container connected to this chamber.

A microscopic filter 25 is inserted between the hybridization chamber 23 and a second compartment 26 of the detection chamber. For example, the microscopic filter 25 will have a cut-off size of approximately 1 micrometer to prevent the solid surfaces from moving inside the detection chamber, while free DNA molecules pass through the filter.

A solvent is preferably introduced in the detection chamber in both compartments. Alternatively, the solvent can be introduced in the injection chamber if the solvent does not affect both the attachment and hybridization chemistry.

The detection chamber is provided with a light source 30 to illuminate the solution contained in both compartments 22 and 26. The light source 30 may be laser light source. In order to facilitate the detection, walls of compartments 22 and 26 should be transparent if the light source 30 is disposed outside the chamber. The detection chamber detects the presence of DNA. If DNA is present in both compartments, that means that the two sequences of the DNA samples which have been compared are not 95 to 100% identical. If DNA is detected in compartment 22 containing the check sample and not in compartment 26, that means that the DNA strands introduced in the hybridization chamber 23 have hybridized to the complementary strand of the reference DNA sample, i.e. the two DNA samples to be compared are identical.

The detection of light scattering or turbidity may be automatic. The device 20 then comprises light sensors 31 and 32 to detect and convert the light intensity into an electric signal. For example, photodiodes are disposed to detect the light intensity in the detection compartments. Preferably, the light intensity is sensed in a direction perpendicular to the incoming ray of light from the light source. This format facilitates the measurement of turbidity and not the opacity. Alternatively, the opacity of the obtained solution can be measured with a sensor disposed in the direct beam of the light source. In this format, a more sensitive sensor is required. In addition, measurements are correlated with eventual variations of the intensity of the light source.

Device 20 is controlled by a central unit 33 (CTRL) that controls not only the light source 30 and sensors 31 and 32, but also valves 34, 35, 36 and 37 interposed in the links between the different chambers/compartments. The control of the different valves is well within the ability of one with an ordinary skill in the art, on the basis of the functional description above.

The invention will be better understood by reference to the following nonlimiting example.

### EXAMPLE

In this example, we used two different DNA solutions (one solution of herring sperm DNA and one solution of random oligonucleotides). 40 microliters of these solutions were added to an equal amount of acetone. After one minute, a laser beam (from a laser module 280-460 from the Farnel Company powered by a standard 9-volts battery) was shined through the glass test tube containing the solution. A photodiode unit (327-646 from the Farnel Company) powered at 20 volts/0.03 ampere was used to record the intensity scattered at 90 degrees. Using an oscilloscope to visualize the output from the photodiode unit, the output voltage went from 17.5 millivolts in the absence of laser beam to 23.5 millivolts when the beam was turned on. This corresponds to a variation of 33% of the output voltage. For a test tube with no DNA, the output voltage did not show any measurable change when the laser was switched on. This demonstrates the possibility to detect the presence of DNA inside the test tube using laser light scattering. In the best case (i.e. with herring sperm DNA), the scattered light was strong enough to be observed with naked eye. Such results have been obtained without any specific precaution to protect the photodiode from the ambient light.

As shown above, the invention is compatible with a small size device. In particular, this is due to the fact that a very small amount of DNA sample is sufficient to be compared to a reference sample. Further, the small element needed to implement the invention participates to obtain such a result.

The amplitude of the variations of the output voltage of the photodiode(s) is large enough, so that standard electronic control equipment can be used to interface with the device according to the present invention. For example, an electronic module turns on a LED to indicate that the DNA solution contains the right type of DNA. Alternatively, the device of the present invention can be connected to a computer in order to record the details of the output signal and to take a decision. To flow the DNA solutions between the different chambers, one can use for example an externally applied pressure (either manually or using a stepping electrical motor). It should be noted that the embodiment illustrated in figure 3 is a functional one.

Having thus described at least one illustrative embodiment of the invention, various alterations, modifications and improvements will readily occur to those skilled in the art. Such alterations, modifications, and improvements are intended to be within the spirit and scope of the invention as claimed. Accordingly, the foregoing description is by way of example only and is not intended to be limiting.

## Claims

1. A method for determining whether or not the sequence of a test DNA molecule and the sequence of a reference DNA molecule are 95 to 100% identical, comprising the steps of:
a) preparing (11) a first solution of single stranded test DNA molecules;
b) attaching a reference DNA molecule (13) to a solid support to form a solid support-reference DNA complex;
c) hybridizing (14) said single stranded test DNA molecules with said solid support-reference DNA complex to form a solid support-reference DNA-test DNA complex wherein said solid support-reference DNA-test DNA complex is formed when the reference DNA and the test DNA sequences are 95 to 100% identical;
d) filtering (15) said solid support-reference DNA-test DNA complex through a filter under conditions such that said test DNA molecules pass through said filter into a filtered solution if said test DNA has not hybridized to said reference DNA, the filter having a cut-off size chosen to prevent the flow of the solid support but allowing the flow of free DNA solution ;
e) adding a solvent (16) to said filtered solution to form solvent treated filtered solution;
f) detecting (18) the presence or absence of said test DNA in said solvent treated filtered solution by measuring the opacity or turbidity or said solvent treated filtered solution wherein the presence of DNA is said filtered solution indicates that the sequences of the test and reference DNA samples were less than 95% identical and wherein the absence of DNA is said filtered solution indicates that the sequences of the test and reference DNA samples were more than 95% identical.

2. The method of claim 1, wherein the solvent (16) is acetone.

3. The method of claim 1, wherein the solvent (16) is ethanol.

4. The method of claim 1 wherein said opacity or turbidity is measured by shining light (17) through said solvent treated filtered solution.

5. The method of claim 4, wherein the light is a laser light.

6. The method of claim 1, wherein the solid support is a suspension of streptavidin-coated microbeads, and the reference DNA molecules include a biotin group.

7. Use of a device (20), comprising:
- an injection chamber (21) arranged to receive the test solution containing single stranded test DNA molecules;
- a hybridization chamber (23) for hybridizing the single stranded test DNA molecules to the reference DNA molecules attached to the solid support to generate the solid support-reference DNA-test DNA complex, an output of the injection chamber being connected to an input of the hybridization chamber;
- a filter (25) having a cut-off size chosen to prevent the flow of the solid support but allowing the flow of free DNA solution, the filter being arranged to filter the content of the hybridization chamber;
- a detection chamber (26) for receiving the content of the hybridization chamber after said filtering; and
- a photodiode unit (31, 32) arranged to record the light intensity scattered by the indicator solution in the detection chamber (26) to detect the presence or absence of said test DNA, in the method of claim 1.

8. The use of claim 7, wherein the device also comprises a light source (30), preferably a laser source, to illuminate the indicator solution contained in the detection chamber (26).

9. The use of any of claims 7 to 8, wherein the detection chamber further comprises a first compartment (26) to contain the indicator solution outputted from the hybridization chamber (23), and a second compartment (22) to contain a control solution of the DNA solution to be tested.

## Patentansprüche

1. Verfahren zum Bestimmen, ob die Sequenz eines Test-DNA-Moleküls und die Sequenz eines Referenz-DNA-Moleküls zu 95 bis 100 % identisch sind oder nicht, das die folgenden Schritte umfasst:
a) Ansetzen (11) einer ersten Lösung aus einsträngigen Test-DNA-Molekülen;
b) Anlagern eines Referenz-DNA-Moleküls (13) an einen Festträger, um einen Festträger-Referenz-DNA-Komplex zu bilden;
c) Hybridisieren (14) der einsträngigen Test-DNA-Moleküle mit dem FestträgerReferenz-DNA-Komplex zum Bilden eines Festträger-Referenz-DNA-TestDNA-Komplexes, wobei der Festträger-Referenz-DNA-Test-DNA-Komplex gebildet wird, wenn die Referenz-DNA- und die Test-DNA-Sequenzen zu 95 bis 100% identisch sind;
d) Filtern (15) des Festträger-Referenz-DNA-Test-DNA-Komplexes durch ein Filter unter Bedingungen, die derart sind, dass die Test-DNA-Moleküle durch das Filter in eine gefilterte Lösung passieren, wenn die Test-DNA nicht an die Referenz-DNA hybridisiert wurde, wobei das Filter eine Trenngrenze aufweist, die ausgewählt ist, um den Fluss des Festträgers zu verhindern, aber den Fluss von freier DNA-Lösung zuzulassen;
e) Beimischen eines Lösungsmittels (16) zu der gefilterten Lösung zum Bilden einer lösungsmittelbehandelten gefilterten Lösung;
f) Detektieren (18) des Vorhandenseins oder Nichtvorhandenseins der Test-DNA in der lösungsmittelbehandelten gefilterten Lösung durch Messen der Opazität oder Trübheit der lösungsmittelbehandelten gefilterten Lösung, wobei das Vorhandensein von DNA in der gefilterten Lösung anzeigt, dass die Sequenzen der Test- und Referenz-DNA-Proben zu weniger als 95% identisch waren, und wobei das Nichtvorhandensein der DNA in der gefilterten Lösung anzeigt, dass die Sequenzen der Test- und Referenz-DNA-Proben zu mehr als 95% identisch waren.

2. Verfahren nach Anspruch 1, wobei das Lösungsmittel (16) Aceton ist.

3. Verfahren nach Anspruch 1, wobei das Lösungsmittel (16) Ethanol ist.

4. Verfahren nach Anspruch 1, wobei die Opazität oder Trübheit mit durch die lösungsmittelbehandelte gefilterte Lösung scheinendes Licht (17) gemessen wird.

5. Verfahren nach Anspruch 4, wobei das Licht ein Laserlicht ist.

6. Verfahren nach Anspruch 1, wobei der Festträger eine Suspension von streptavidinbeschichteten Mikrokügelchen ist und die Referenz-DNA-Moleküle eine Biotin-Gruppe umfassen.

7. Verwendung einer Vorrichtung (20), die Folgendes umfasst:
- eine Injektionskammer (21), die eingerichtet ist, um die Testlösung aufzunehmen, die einsträngige Test-DNA-Moleküle enthält,
- eine Hybridisierungskammer (23) zum Hybridisieren der einsträngigen Test-DNA-Moleküle an die Referenz-DNA-Moleküle, die an dem Festträger anlagern, um den Festträger-Referenz-DNA-Test-DNA-Komplex zu erzeugen, wobei ein Ausgang der Injektionskammer mit einem Eingang der Hybridisierungskammer verbunden ist;
- ein Filter (25), das eine Trenngrenze aufweist, die ausgewählt ist, um den Fluss des Festträgers zu verhindern, aber den Fluss von freier DNA-Lösung zuzulassen, wobei das Filter eingerichtet ist, um den Inhalt der Hybridisierungskammer zu filtern;
- eine Detektionskammer (26) zum Aufnehmen des Inhalts der Hybridisierungskammer nach dem Filtern; und
- eine Photodiodeneinheit (31, 32), die eingerichtet ist, um die Stärke des Lichts aufzuzeichnen, das durch die Indikatorlösung in der Detektionskammer (26) gestreut wird, um das Vorhandensein oder Nichtvorhandensein der Test-DNA in dem Verfahren nach Anspruch 1 festzustellen.

8. Verwendung nach Anspruch 7, wobei die Vorrichtung auch eine Lichtquelle (30), vorzugsweise eine Laserquelle, umfasst, um die Indikatorlösung, die in der Detektionskammer (26) enthalten ist, zu beleuchten.

9. Verwendung nach einem der Ansprüche 7 bis 8, wobei die Detektionskammer überdies eine erste Kammer (26), um die Indikatorlösung zu enthalten, die von der Hybridisierungskammer (23) ausgegeben wird, und eine zweite Kammer (22) umfasst, um eine Kontrolllösung der zu testenden DNA-Lösung zu enthalten.

## Revendications

1. Procédé pour déterminer si la séquence d'une molécule d'ADN à tester et la séquence d'une molécule d'ADN de référence sont identiques de 95 à 100 %, comprenant les étapes suivantes :
a) préparer (11) une première solution de molécules d'ADN à simple brin à tester ;
b) attacher une molécule d'ADN de référence (13) à un support solide pour former un complexe support solide - ADN de référence ;
c) hybrider (14) les molécules d'ADN à simple brin à tester avec le complexe support solide - ADN de référence pour former un complexe support solide - ADN de référence - ADN de test, le complexe support solide - ADN de référence - ADN de test étant formé lorsque les séquences d'ADN de référence et d'ADN de test sont identiques de 95 à 100 % ;
d) filtrer (15) le complexe support solide - ADN de référence - ADN de test par un filtre dans des conditions telles que les molécules d'ADN de test passent à travers le filtre dans une solution filtrée si l'ADN de test ne s'est pas hybridé avec l'ADN de référence, le filtre ayant une taille de coupure choisie de façon à empêcher le passage du flux de support solide mais permettant le passage du flux de solution d'ADN libre ;
e) ajouter un solvant (16) à la solution filtrée pour former une solution filtrée traitée par solvant ;
f) détecter (18) la présence ou l'absence de l'ADN de test dans la solution filtrée traitée par solvant en mesurant l'opacité ou la turbidité de la solution filtrée traitée par solvant, la présence d'ADN dans la solution filtrée indiquant que les séquences des échantillons d'ADN de test et de référence sont identiques à moins de 95 %, et l'absence d'ADN dans la solution filtrée indiquant que les séquences des échantillons d'ADN de test et de référence sont identiques à plus de 95 %.

2. Procédé selon la revendication 1, dans lequel le solvant (16) est de l'acétone.

3. Procédé selon la revendication 1, dans lequel le solvant (16) est de l'éthanol.

4. Procédé selon la revendication 1, dans lequel l'opacité ou la turbidité est mesurée par de la lumière brillante (17) à travers la solution filtrée traitée par solvant.

5. Procédé selon la revendication 4, dans lequel la lumière est de la lumière laser.

6. Procédé selon la revendication 1, dans lequel le support solide est une suspension de microbilles revêtues de streptavidine, et les molécules d'ADN de référence comprennent un groupe biotine.

7. Utilisation d'un dispositif (20), comprenant .
- une chambre d'injection (21) agencée pour recevoir la solution contenant des molécules d'ADN à simple brin à tester ;
- une chambre d'hybridation (23) pour hybrider les molécules d'ADN à simple brin à tester avec les molécules d'ADN de référence attachée au support solide pour générer le complexe support solide - ADN de référence - ADN de test, et la sortie de la chambre d'injection étant connectée à une entrée de la chambre d'hybridation ;
- un filtre (25) ayant une taille de coupure choisie de façon à empêcher le passage du flux de support solide mais permettant le passage du flux de solution d'ADN libre, le filtre étant agencé pour filtrer le contenu de la chambre d'hybridation ;
- une chambre de détection (26) pour recevoir le contenu de la chambre d'hybridation après le filtrage ; et
- un module de photodiodes (31, 32) agencé pour enregistrer l'intensité lumineuse diffusée par la solution indicatrice dans la chambre de détection (26) pour détecter la présence ou l'absence de l'ADN de test, dans le procédé de la revendication 1.

8. Utilisation selon la revendication 7, dans laquelle le dispositif comprend aussi une source de lumière (30), de préférence une source laser, pour éclairer la solution indicatrice contenue dans la chambre de détection (26).

9. Utilisation selon l'une quelconque des revendications 7 à 8, dans laquelle la chambre de détection comprend en outre un premier compartiment (26) pour contenir la solution indicatrice obtenue à partir de la chambre d'hybridation (23), et un deuxième compartiment (22) pour contenir une solution de contrôle de la solution d'ADN à tester.
